# EUROPÄISCHE PATENTANMELDUNG

(11) **EP 2 287 586 A2**
(43) Veröffentlichungstag der Anmeldung: **23.02.2011**
(21) Anmeldenummer: 10194392.6
(22) Anmeldetag: 07.07.2004
(51) Int. Cl.: G01L 19/08, G01D 3/02, A61B 5/00, H01L 23/48

(54) **Integrierte Sensor-Chip-Einheit**

(30) Priorität: 19.07.2003 DE 10332878
(62) Teilanmeldung aus: 04766151.7
(71) Anmelder: Infineon Technologies AG, 85579 Neubiberg (DE)
(72) Erfinder: Bauer, Robert, 85665, Moosach (DE); von Hagen, Jochen, 83059, Kolbermoor (DE)
(74) Vertreter: Karl, Frank

(57) **Zusammenfassung**

Die Erfindung betrifft einen Sensorbaustein, insbesondere einen Messwertaufnehmer (2) zur Ermittlung von Messdaten und eine Schaltungsanordnung (3) zum Ermöglichen einer drahtloser Energieversorgung und Abfrage der Messdaten. Der Messwertaufnehmer ist als integrationsfähiger Sensor (2) ausgebildet, und die Schaltungsanordnung als integrierter Halbleiterschaltungsbaustein (3), wobei der Sensor (2) und der Halbleiterschaltungsbaustein (3) mit Mitteln der Mikrosystemtechnik mechanisch und elektrisch leitend miteinander zu einer integrierten Sensor-Chip-Einheit (1)verbunden sind.

## Beschreibung

### Beschreibung

### Integrierte Sensor-Chip-Einheit

Die vorliegende Erfindung bezieht sich auf einen Sensorbaustein, insbesondere auf Messwertaufnehmer zur Ermittlung von Messdaten und eine Schaltungsanordnung zum Ermöglichen einer drahtloser Energieversorgung und Abfrage der Messdaten gemäß dem Oberbegriff des Anspruchs 1.

Derartige Sensoren sind bekannt. Aus dem deutschen Gebrauchsmuster DE 201 21 388 U1 ist ein Sensor bekannt, bei dem eine deformierbare Membrane und eine Gegenelektrode mikrostrukturiert sind und eine Kapazität eines Schwingkreises bilden. Eine Auswerteschaltung ist in dem Sensor nicht integriert.

Im deutschen Gebrauchsmuster DE 202 02 131 U1 ist ein System zur kontinuierlichen Überwachung von biometrischen Daten eines Lebewesens beschrieben. Dort sind ein Sensor mit einem Transponder und eine von der Auswerteeinheit separate Abfragestation vorgesehen. Der Sensor weist dabei keine Auswerteschaltung auf.

In der DE 43 41 903 A1 ist ferner eine Vorrichtung beschrieben, die zur kontinuierlichen Messung des Druckes und/oder des Durchflusses und/oder der Temperatur und/oder von Potentialen und Strömen in Körpern oder Organen von Menschen und Tieren geeignet ist. Die dort beschriebene Vorrichtung übermittelt die Messwerte bzw. Messsignale ohne Verkabelung perkutan (d.h. durch die Haut hindurch) an eine außerhalb des Körpers befindliche Empfängereinheit, welche die Messsignale verarbeitet und zur Anzeige bringt.

Ferner ist aus der amerikanischen Patentschrift US 5 711 861 ein elektrochemischer Sensor bekannt, dessen Signale über einen Transmitter drahtlos zu einem externen Empfänger übertragen und mittels Computer ausgewertet werden Derartige Sensoren die kabellos arbeiten, und bei denen sowohl die Messwertauslesung als auch die Energieversorgung drahtlos erfolgt, sind also prinzipiell bereits bekannt.

Da die bekannten Sensoren keine Aufbereitungsschaltungen beinhalten, weil sie dann zu große Volumina einnehmen würden, kann es zu Störungen bei der Signalübertragung kommen. Für die schwachen Signale der Messwertaufnehmer wird die Verstärkung und Aufbereitung erst außerhalb des Körpers möglich, weil erst hier die entsprechenden Schaltungen zur Verfügung stehen. Komplexe Auswerteschaltungen im Körper von Patienten zu implantieren wäre für die Patienten aufgrund der großen Volumina unangenehm und schwierig durchzuführen.

Der vorliegenden Erfindung liegt die Aufgabe zu Grunde, einen Messwertaufnehmer zu schaffen, der bereits eine Auswerteelektronik enthält, damit die erzeugten Messsignale bereits aufbereitet drahtlos übertragen werden können. Dabei sollte die bestimmende Größe der Sensor sein.

Diese Aufgabe wird mit einem Messwertaufnehmer gelöst, welcher die Merkmale des Anspruchs 1 aufweist. Vorteilhafte Ausgestaltungen entnimmt man den abhängigen Ansprüchen.

Die Vorteile des erfindungsgemäßen Messwertaufnehmers liegen in seiner geringen Größe, seiner Zuverlässigkeit und in der Möglichkeit, bereits digitalisierte Messsignale zu übertragen. Dadurch sinkt die Störanfälligkeit und die Zuverlässigkeit bei der Messwertgewinnung steigt in dem selben Maße an.

Ein gemäß der vorliegenden Erfindung gestalteter Messwertaufnehmer zur Ermittlung von Messdaten und eine Schaltungsanordnung zum Ermöglichen einer drahtlosen Energieversorgung und Abfrage der Messdaten sind besonders vorteilhaft, wenn der Messwertaufnehmer als integrationsfähiger Sensor ausgebildet ist, und wenn die Schaltungsanordnung als integrierter Halbleiterschaltungsbaustein ausgeführt ist, wobei der Sensor und der Halbleiterschaltungsbaustein mit Mitteln der Mikrosystemtechnik mechanisch und elektrisch leitend miteinander zu einer integrierten Sensor-Chip-Einheit verbunden sind.

Besonders vorteilhaft ist ein Messwertaufnehmer, wenn die Mittel der Mikrosystemtechnik beispielsweise die "Flip-Chip-Technik" als Verfahren beinhalten.

Vorteilhafte Messwertaufnehmer sind zu realisierten, wenn der Sensor durch einen biometrischen Sensor, beispielsweise einen Glucose-Sensor realisiert wird. Auch ein Blutsauerstoff-Sensor oder ein Lichtabsorbtions-Sensor kann bei der Erfindung vorteilhaft eingesetzt werden.

Ebenso ist es vorstellbar, dass der Sensor durch einen Druck-Sensor, einen Thermo-Sensor oder einen chemischen Sensor realisiert wird.

Ferner ist es vorteilhaft, wenn der Halbleiterschaltungsbaustein Bauelemente zur Energieversorgung und eine Messwertaufbereitungsschaltung, insbesondere mit Digitalisierungsstufe zum Datenaustausch aufweist.

Weitere Einsatzgebiete ergeben sich, wenn die integrierte Sensor-Chip-Einheit von einer Kapselung umgeben ist.

Derartige Einsatzgebiete sind beispielsweise Implantationen in menschliche oder tierische Körper. Ferner ist es möglich, dass die integrierte Sensor-Chip-Einheit aufgrund ihrer Kapselung in Körperflüssigkeiten schwimmt.

Darüber hinaus ist es vorteilhaft, dass die Energieversorgung und der Austausch der Daten durch eine fernwirkende Empfangseinheit erfolgt, insbesondere, wenn die Fernwirkung auf induktiver Kopplung basiert.

In der allgemeinen Technologie ist eine integrierte Sensor-Chip-Einheit sehr gut in Wandungen eines Rohrleitungssystems oder anderen Behältern integrierbar.

Ebenso vorteilhaft lassen sich integrierte Sensor-Chip-Einheiten zur Prozessüberwachung in Behältern mit Flüssigkeiten einsetzen.

In den folgenden Zeichnungen wird die Erfindung anhand von Ausführungsbeispielen noch näher erläutert.

Es zeigt
Figur 1 einen stark schematisierten Sensor-Chip gemäß der Erfindung;
Figur 2 einen weiteren Sensor-Chip gemäß der Erfindung in Seitenansicht;
Figur 3 die Draufsicht auf einen Sensor-Chip gemäß Figur 2, stark schematisiert;
Figur 4 ein weiteres Ausführungsbeispiel eines Sensor-Chips in Seitenansicht und
Figur 5 eine weitere Variante einer Anwendung eines Sensors-Chips gemäß der Erfindung.

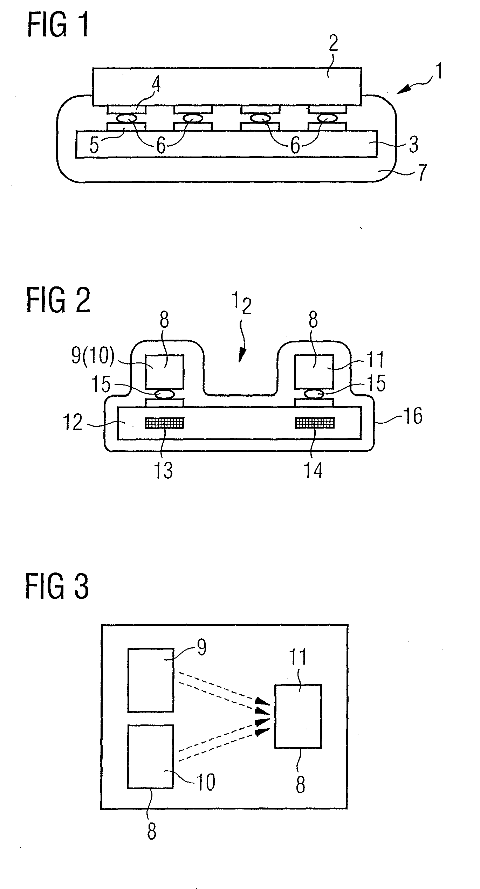

In Figur 1 ist eine integrierte Sensor-Chip-Einheit 1 dargestellt, die zur Bestimmung des Glucose-Gehalts im Blut eines Lebewesens dient. Zur Messung des Glucose-Gehalts im Blut sind - wie eingangs schon erläutert, druckschriftlich zitiert und beschrieben - prinzipiell bereits derartige Sensoren 2 bekannt. Sie basieren z.B. auf Kapazitätsmessungen. Um die auf diese Weise erzeugten Signale aufzubereiten ist eine komplexe Schaltungsanordnung erforderlich. Eine dazu notwendige Elektronik lässt sich durch Verwendung von integrierten Schaltkreisen sehr klein gestalten und mündet letztlich in einem integrierten Schaltkreis in Form eines integrierten Halbleiterschaltungsbausteins 3. Mit Mitteln der Mikrosystemtechnik sind der Sensor 2 und der integrierte Halbleiterschaltungsbaustein 3 miteinander verbunden. Diese Verbindung erfolgt elektrisch und mechanisch, so dass eine integrierte Sensor-Chip-Einheit 1 gemäß der Erfindung entsteht.

Der Messwertaufnehmer 2 ist in so genannter Nano-Technologie ausgeführt und benötigt ebenso wie der integrierte Halbleiterschaltungsbaustein 3 nur ein sehr geringes Volumen.

Als Beispiel für die Verbindung des Messwertaufnehmers 2 mit dem integrierten Halbleiterschaltungsbaustein 3 ist eine Verbindung nach Art einer so genannten Flip-Chip Methode gezeigt.

Dies ist eine Technologie bei der Halbleiterchips auf denen sich Lotkügelchen (Bumps) befinden direkt ohne eigenes Gehäuse auf die Platine montiert werden. Um das bestückte Bauteil auf der Platine zu fixieren wird nur ein Flussmittel aufgebracht, das zunächst als Klebstoff wirkt und beim folgenden Lötprozess wieder verdampft. Die Bezeichnung Flip-Chip rührt daher, dass der Chip mit den Lotkügelchen nach oben liegt und vor dem Bestücken gedreht (geflippt) werden muss. Erläutert wird diese Technik beispielsweise auf der Internetseite des Unternehmens "Binder Elektronik GmbH" http://www.binder-elektronik.de/.

Es können aber auch andere Technologien der Mikrosystemtechnik Anwendung finden. Eine weiteres Verfahren ist das so genannte Ball-Wedge-Bonding, damit können Gold-Draht-Bondverbindungen hergestellt werden und somit die integrationsfähigen Bauelemente in ChipOnBoard-Technologie (COB) aufgebaut werden.

Ferner besteht die Möglichkeit so genannte Gold-Stud-Bumps auf Silizium-Träger zu setzen. Diese Stud-Bump-Flip-Chips können dann mit leitfähigen bzw. Snap-Cure-Klebern montiert werden. Dies ist besonders ideal für Kleinserien.

Bei der Erfindung wird nun diese Mikrosystemtechnik direkt so angewendet, dass ein Messwertaufnehmer (Sensor 2) mit Hilfe dieser Technologie nicht auf einer Platine, sondern mit dem integrierten Halbleiterschaltungsbaustein 3 direkt verbunden wird, welcher die gesamte Auswerteelektronik für den Messwertaufnehmer (Sensor 2) enthält. Durch diesen weiteren Integrationsschritt kann das Volumen der integrierte Sensor-Chip-Einheit noch weiter deutlich verringert werden.

Um die Verbindung herzustellen enthalten sowohl der Sensor 2 als auch der Halbleiterschaltungsbaustein 3 Anschlussflächen in Form von so genannten "Pads" 4 und 5, mit deren Hilfe eine innige metallische Verbindung zwischen den Bauelementen - Sensor 2 und Halbleiterschaltungsbaustein 3 - geschaffen werden kann. Dazu sind jeweils zwischen diesen Pads 4 und 5 Lötkügelchen 6 angeordnet. Beim Zusammenfügen von Sensor 2 und Halbleiterschaltungsbaustein 3 gemäß der Mikrosystemtechnik schmelzen diese Lötkügelchen 6 beispielsweise unter dem Einfluss von Wärme und Druck, so dass zwischen Sensor 2 und Halbleiterschaltungsbaustein 3 eine innige metallische Verbindung geschaffen wird. Die Einheit "Messwertaufnehmer/integrierter Halbleiterschaltungsbaustein" 2/3 bildet somit nach dem Vereinigungsvorgang eine integrierte Sensor-Chip-Einheit 1, die mit einer Kapselung 7 versehen ist, so dass sie in einen tierischen oder menschlichen Körper implantiert werden kann.

In Figur 2 ist eine Seitenansicht eines weiteren Ausführungsbeispiels für eine erfindungsgemäße integrierte Sensor-Chip-Einheit 1₂ dargestellt und in der Figur 3 ist die zugehörige Draufsicht dieses stark schematisierten Ausführungsbeispiels zu sehen.

Dort ist ein Sensor 8 gezeigt, mit dessen Hilfe die Konzentration des Sauerstoffgehalts im Blut eines Lebewesens gemessen werden kann. Der Messwertaufnehmer (Sensor 8) weist wenigstens zwei Leuchtdioden 9 und 10 auf. Die Leuchtdioden 9 und 10 emittieren Licht verschiedener Spektren, z. B. arbeitet eine im roten und die andere im infraroten Bereich. Ferner trägt der Messwertaufnehmer (Sensor 8) eine Photodiode 11, die Lichtemissionen der Leuchtdioden 9 und 10 empfängt. Die Auswerteelektronik befindet sich analog zum Ausführungsbeispiel gemäß Figur 1 wieder in einem integrierten Halbleiterschaltungsbaustein 12, der in vorteilhafter Weise auch Elemente wie beispielsweise Spulen 13 und 14 zur Energie und Datenübertragung aufweist. Auch diese in den Figuren 2 und 3 gezeigte integrierte Sensor-Chip-Einheit 1₂, bestehend aus einem Messwertaufnehmer 8 und einem Halbleiterschaltungsbaustein 12, ist - ähnlich wie die integrierte Sensor-Chip-Einheit 1 gemäß dem Ausführungsbeispiel nach der Figur 1 - mit Mitteln der aus der Mikrosystemtechnik bekannten Flip-Chip-Technologie mittels Lötkügelchen 15 zusammengefügt und mit einer Kapselung 16 versehen, so dass sie eine äußerst geringe Größe aufweist und in einem Lebewesen ohne weiteres implantiert werden kann.

Bei beiden Ausführungsbeispielen enthält der Halbleiterschaltungsbaustein 3 bzw. 12 Spulen zur Energie und Datenübertragung, so dass die Messdaten der integrierten Sensor-Chip-Einheit 1 bzw. 1₂ berührungslos von außerhalb des Körpers des Lebewesens abgefragt werden können. Diese berührungslose Übertragung von Messdaten ist an sich bekannt, wird aber durch die geringe Größe der erfindungsgemäßen integrierten Sensor-Chip-Einheit noch einfacher und vor allem zuverlässiger möglich, da sich die Aufbereitungsschaltung für die Messdaten bereits im Halbleiterschaltungsbaustein 3 bzw. 12 befinden, welcher erfindungsgemäß Bestandteil der integrierten Sensor-Chip-Einheit 1 bzw. 1₂ ist.

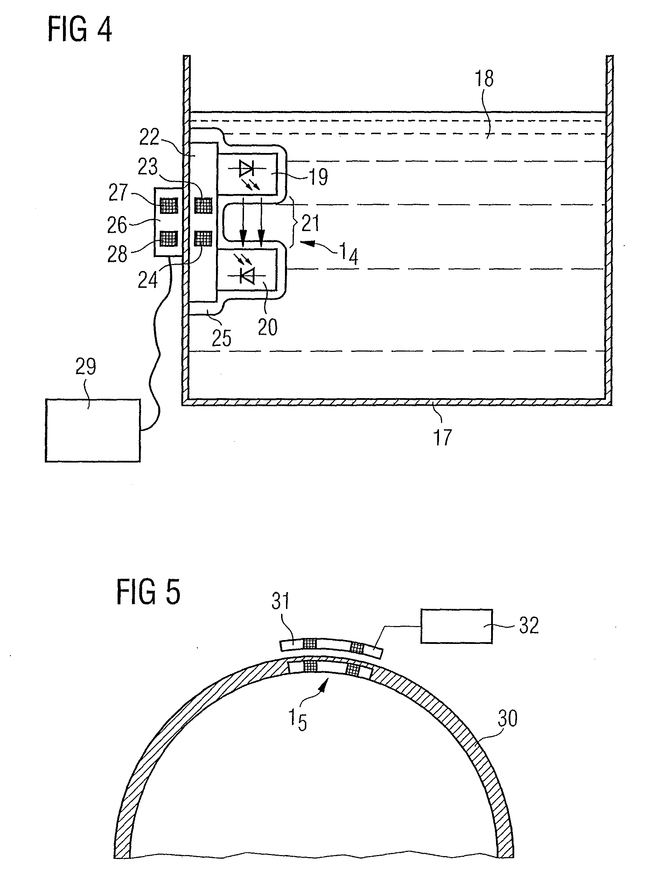

Ein weiteres Ausführungsbeispiel ist in der Figur 4 dargestellt. In einem Behälter 17 befindet sich eine Flüssigkeit 18, deren chemische Reaktion überwacht werden soll. Um den Fortgang des Prozesses zu verfolgen muss die Konzentration des Stoffes und dessen Verteilung in dem Behälter 17 bestimmt werden. Die Konzentration lässt sich mit Hilfe von Lichtabsorbtion der Flüssigkeit 18 bestimmen. Um die Lichtabsorbtion zu ermitteln weist eine integrierte Sensor-Chip-Einheit 1₄ wenigstens eine Leuchtdiode 19 bestimmter Wellenlänge auf. Die emittierte Strahlung der Leuchtdiode 19 wird von einer Photodiode 20 empfangen. Zwischen der emittierenden Leuchtdiode 19 und der empfangenden Photodiode 20 befindet sich ein Zwischenraum, in welchem die zu überwachende Flüssigkeit 18 zirkuliert. Der Zwischenraum kann als Absorptionsstrecke 21 bezeichnet werden, innerhalb derer die zirkulierende Flüssigkeit 18 gemessen wird.

Die Leuchtdiode 19 und die Photodiode 20 sind in der bereits zu den anderen Ausführungsbeispielen beschriebenen Weise mit Hilfe der Mikrosystemtechnik auf einem Halbleiterschaltungsbaustein 22 integriert, welcher die elektronische Aufbereitungsschaltung sowie zwei Spulen 23 und 24 für die Energieversorgung und den Datenaustausch enthält. Der integrierte Halbleiterschaltungsbaustein 22 steuert u.a. die LEDs (Leuchtdiode 19 und Photodiode 20) an, bereitet die Messwerte auf und digitalisiert sie. Von den in dem Halbleiterschaltungsbaustein 22 integrierten Spulen 23 und 24 dient eine - Spule 23 - der Energieübertragung, die andere - Spule 23 - dem Datenaustausch und der Programmierung des Halbleiterschaltungsbausteins 22. Die gesamte integrierte Sensor-Chip-Einheit 1₄ ist mit einer Kapselung 25 versehen, so dass sie nicht von den Substanzen der im Behälter 17 befindlichen Flüssigkeit 18 angegriffen werden kann.

Von der integrierten Sensor-Chip-Einheit 1₄ können mehrere an unterschiedlichen Orten der Innenwandung des Behälters 17 angeordnet sein. An der Außenseite des Behälters 17 können eine oder mehrere Empfangseinheiten 26 angebracht sein. Die Empfangseinheiten 26 arbeiten drahtlos und können die integrierten Sensor-Chip-Einheiten 1₄ parallel oder seriell auslesen. Sie enthalten ihrerseits zwei Spulen 27 und 28, von denen eine - Spule 27 - der Energieübertragung dient und die zweite - Spule 28 - dem Datenaustausch. An die Empfangseinheit 26 ist ein Auswertegerät, beispielsweise ein Computer 29 angeschlossen. An den Einbauorten für die integrierten Sensor-Chip-Einheiten 1₄ muss der Behälter 17 aus nichtmetallischem Werkstoff bestehen.

In Figur 5 ist ein weiteres Ausführungsbeispiel dargestellt, in welchem der Einbau einer erfindungsgemäßen integrierten Sensor-Chip-Einheit 1₅ demonstriert ist. Ein Rohrleitungssystem 30 dient zur Versorgung mit Druckluft oder einem Prozessgas. Durch Erhöhung der Anzahl von Verbrauchsstellen im System kann es zur Reduzierung des Drucks kommen, oder es kommt zu Druckverlusten durch Undichtigkeiten im System. Diese zu suchen erweist sich oftmals als sehr schwierig und aufwendig, da jede Verbrauchstation manuell untersucht werden muss. Durch die Installation von mehreren Drucksensoren lässt sich die Problemstelle im System schneller eingrenzen. Die Bauelemente entsprechen denen aus den vorbeschriebenen Ausführungsbeispielen, allerdings ist die integrierte Sensor-Chip- Einheit 1₅ als Drucksensor mit integrierter Auswerteschaltung gemäß den vorbeschriebenen Ausführungsbeispielen ausgebildet. Eine Empfangseinheit 31 mit einem Computer als Auswertegerät 32 lässt sich an dem Rohrleitungssystem 30 entlang führen, um die Messwerte der jeweiligen integrierten Sensor-Chip-Einheiten 1₅ aus zu lesen.

Die integrierten Sensor-Chip-Einheiten können, wie im Ausführungsbeispiel gemäß der Figur 5 gezeigt, in der Rohrwandung bereits bei dessen Herstellung eingebracht werden. Dadurch erhöhen sich die Kosten der Rohre nur unwesentlich, da die Herstellungskosten der integrierten Sensor-Chip-Einheiten deutlich unter den ohnehin anfallenden Herstellungskosten der Rohre liegen. Aber auch eine separate Befestigung der integrierten Sensor-Chip-Einheiten an den Innenwänden der Rohre ist mit Vorteil zu realisieren. In allen Anwendungsfällen lassen sich die integrierten Sensor-Chip-Einheiten in bestimmten Abständen im Rohr anordnen, so dass die Auswerteeinheit an der Rohraußenwandung entlang geführt werden kann, um sukzessive nach einander alle in Frage kommenden integrierten Sensor-Chip-Einheiten auszulesen.

### Bezugszeichenliste

- 1: integrierte Sensor-Chip-Einheit
- 1₂: integrierte Sensor-Chip-Einheit
- 1₄: integrierte Sensor-Chip-Einheit
- 1₅: integrierte Sensor-Chip-Einheit
- 2: Messwertaufnehmer (Sensor)
- 3: Halbleiterschaltungsbaustein
- 4: Pad
- 5: Pad
- 6: Lötkügelchen (Bump)
- 7: Kapselung
- 8: Messwertaufnehmer (Sensor)
- 9: Leuchtdiode
- 10: Leuchtdiode
- 11: Photodiode
- 12: Halbleiterschaltungsbaustein
- 13: Spule
- 14: Spule
- 15: Lötkügelchen
- 16: Kapselung
- 17: Behälter
- 18: Flüssigkeit
- 19: Leuchtdiode
- 20: Photodiode
- 21: Absorptionsstrecke
- 22: Halbleiterschaltungsbaustein
- 23: Spule
- 24: Spule
- 25: Kapselung
- 26: Empfangseinheit
- 27: Spule
- 28: Spule
- 29: Computer
- 30: Rohrleitungssystem
- 31: Empfangseinheit
- 32: Computer

## Patentansprüche

1. Messwertaufnehmer zur Ermittlung von Messdaten und Schaltungsanordnung zum Ermöglichen einer drahtlosen Energieversorgung und Abfrage der Messdaten, **dadurch gekennzeichnet, dass** der Messwertaufnehmer als integrationsfähiger Sensor (2, 8, 19, 20) ausgebildet ist, und dass die Schaltungsanordnung als integrierter Halbleiterschaltungsbaustein (3, 12, 22) ausgeführt ist, wobei der Sensor (2, 8, 19, 20) und der Halbleiterschaltungsbaustein (3, 12, 22) mit Mitteln der Mikrosystemtechnik mechanisch und elektrisch leitend miteinander zu einer integrierten Sensor-Chip-Einheit (1, 1₂, 1₄, 1₅) verbunden sind.

2. Messwertaufnehmer nach Anspruch 1, **dadurch gekennzeichnet, dass** die Mittel der Mikrosystemtechnik Verfahren wie die "Flip-Chip-Technik" umfassen.

3. Messwertaufnehmer nach einem der vorstehenden Ansprüche, **dadurch gekennzeichnet, dass** der Sensor durch einen Lichtabsorbtions-Sensor (8, 19, 20) realisiert wird.

4. Messwertaufnehmer nach einem der vorstehenden Ansprüche, **dadurch gekennzeichnet, dass** der Sensor durch einen Druck-Sensor realisiert wird.

5. Messwertaufnehmer nach einem der vorstehenden Ansprüche, **dadurch gekennzeichnet, dass** der Sensor durch einen Thermo-Sensor realisiert wird.

6. Messwertaufnehmer nach einem der vorstehenden Ansprüche, **dadurch gekennzeichnet, dass** der Sensor durch einen chemischen Sensor realisiert wird.

7. Messwertaufnehmer nach einem der vorstehenden Ansprüche, **dadurch gekennzeichnet, dass** der Halbleiterschaltungsbaustein (3, 12, 22) Bauelemente (13, 14; 23, 24) zur Energieversorgung und zum Datenaustausch aufweist.

8. Messwertaufnehmer nach einem der vorstehenden Ansprüche, **dadurch gekennzeichnet, dass** der Halbleiterschaltungsbaustein (3, 12, 22) eine Messwertaufbereitungsschaltung aufweist.

9. Messwertaufnehmer nach einem der vorstehenden Ansprüche, **dadurch gekennzeichnet, dass** der Halbleiterschaltungsbaustein (3, 12, 22) eine Messwertaufbereitungsschaltung mit Digitalisierungsstufe aufweist.

10. Messwertaufnehmer nach einem der vorstehenden Ansprüche, **dadurch gekennzeichnet, dass** die integrierte Sensor-Chip-Einheit (1, 1₂, 1₄, 1₅) von einer Kapselung (7, 16, 25) umgeben ist.

11. Messwertaufnehmer nach einem der vorstehenden Ansprüche, **dadurch gekennzeichnet, dass** die Energieversorgung und der Austausch der Daten durch eine fernwirkende Empfangseinheit (26, 31) erfolgt.

12. Messwertaufnehmer nach einem der vorstehenden Ansprüche, **dadurch gekennzeichnet, dass** die Fernwirkung auf induktiver Kopplung basiert.

13. Messwertaufnehmer nach einem der vorstehenden Ansprüche, **dadurch gekennzeichnet, dass** er in Wandungen eines Rohrleitungssystems (30) integriert ist.

14. Messwertaufnehmer nach einem der vorstehenden Ansprüche, **dadurch gekennzeichnet, dass** der Sensor durch einen Druck-Sensor realisiert wird und dass der Sensor in einer Rohrwandung bei der Herstellung eines Rohrs eingebracht ist oder dass er an den Innenwänden eines Rohrs separat befestigt ist.

15. Messwertaufnehmer nach Anspruch 14, **dadurch gekennzeichnet, dass** eine Auswerteeinheit zum Auslesen an der Rohraußenwandung entlang geführt wird.

16. Messwertaufnehmer nach einem der Ansprüche 1 bis 12, **dadurch gekennzeichnet, dass** er in Wandungen eines Behälters (17) integriert ist.

17. Messwertaufnehmer nach Anspruch 16, **dadurch gekennzeichnet, dass** der Sensor durch einen Lichtabsorbtions-Sensor (8, 19, 20) realisiert wird.

18. Messwertaufnehmer nach Anspruch 16 oder 17, **dadurch gekennzeichnet, dass** an der Außenseite des Behälters eine Empfangseinheit (26) angebracht ist.

19. Messwertaufnehmer nach einem der Ansprüche 16 bis 18, **dadurch gekennzeichnet, dass** der Behälter eine Flüssigkeit enthält, deren chemische Reaktion überwacht wird.

20. Messwertaufnehmer nach einem der Ansprüche 16 bis 19, **dadurch gekennzeichnet, dass** mehrere Messwertaufnehmer an unterschiedlichen Orten der Innenwandung des Behälters (17) angeordnet sind.
